# EUROPEAN PATENT APPLICATION

(11) **EP 1 319 954 A1**
(43) Date of publication of application: **18.06.2003**
(21) Application number: 01403216.3
(22) Date of filing: 12.12.2001
(51) Int. Cl.: G01N 33/68, C07K 17/00

(54) **Methods for protein analysis using protein capture arrays**

(71) Applicant: Centre National de Genotypage, 91000 Evry (FR)
(72) Inventor: Gut, Yvo Glynne, 75014 Paris (FR)
(74) Representative: Warcoin, Jacques

(57) **Abstract**

The invention relates to a method allowing identification and/or quantifying and/or characterizing proteins in a protein mixture, wherein the proteins are stratified on feature(s) on an array, and a procedure, preferably mass spectrometric analysis, is applied on the proteins on the feature(s), allowing determination of the nature and quantities of the proteins. In particular, the method allows the comparative analysis of nature and amount of proteins in at least two samples. It also allows the targeted selection of proteins out of a mixture of proteins. It further identifies three-dimensional structures that can interact with a selected target protein or a modification of said protein.

## Description

The invention relates to a method allowing identification and/or quantifying and/or characterizing proteins in a protein mixture, wherein the proteins are stratified on feature(s) on an array, and a procedure, preferably a mass spectrometric analysis, is applied to the proteins on the feature(s), allowing determination of the nature and quantities of the proteins. In particular, the method allows the discriminative analysis of interactions of proteins with a three-dimensional structure. It also allows the targeted selection of proteins out of a mixture of proteins. It further identifies three-dimensional structures that can interact with a selected target protein or a modification of said protein.

The term "target" refers to a protein molecule that has an affinity for a given compound on a feature. A target can be employed in its unaltered state (preferably with no alteration of the 3-dimensional structure of the protein). Targets may also be modified.

In preferred embodiments, they harbor a fluorescent or radioactive moiety, or groups or isotopes that can be identified by mass spectrometry. In specific embodiments, targets are labeled, wherein said labeling consists in a chemical modification of the proteins, preferably said chemical modification does not alter the 3-dimensional structure of the protein.

In some embodiments, said chemical modification consists of attaching a chemical group chosen in the group consisting of trinitrobenzene sulfonic acid, ethylthiofluoro acetate, succinic anhydride, phenylisothiocyanate, Dansyl chloride, acetic anhydride, polyethylene glycol, and similar reagents to the (deprotected) N-terminal group of the protein.

In other embodiments, said chemical modification consists of inducing SH-specific protein modifications with an agent chosen in the group consisting of β-mercatoethanol, dithiothreitol, iodoacetic acid, iodoacetamide, and the like.

In yet another embodiment, said chemical modification consists of modifying carboxyl groups of proteins by full or limited amidation using an agent chosen from the group consisting of 1-ethyl-3-[3-(dimethylamino)propyl]carboiimide hydrochloride (EDC), an amine like glycine methyl ester, glycinamide, methylamine, ethanolamine and the like.

In yet another embodiment, said chemical modification consists of a modification of tyrosines by nitration with tetranitromethane (also oxidation of thiols), or of tryptophans by specific oxidation, for example by N-bromosuccinimide, or by limited oxidation with ozone.

A "feature" according to the invention is defined as an area of a substrate having a collection of same-nature, surface-immobilized molecules. One feature is different than another feature if the molecules of the different features have a different structural formula and/or 3-dimensional conformation.

The term "array" refers to a substrate having a two-dimensional surface having at least two different features. Arrays are preferably ordered so that the localization of each feature on the surface is defined. In preferred embodiments, an array can have a density of at least five hundred, at least one thousand, at least 10 thousand, at least 100 thousand features per square cm. The substrate can be, merely by way of example, glass, silicon, quartz, polymer, plastic or metal and can have the thickness of a glass microscope slide or a glass cover slip. Substrates that are transparent to light are useful when the method of performing an assay on the chip involves optical detection. The substrate may also be a membrane made of polyester or nylon. In this embodiment, the density of features per square cm is comprised between a few units to a few dozens.

Preparation of arrays and features is described below.

The term "distinguishable phenotype" has to be understood as a phenotype (i.e. a qualitative or quantitative measurable feature of an organism) that can allow the categorization of a given population. For example, a distinguishable phenotype encompasses the membership to a set of a given disease, or a peculiar feature or property (e.g. resistance or adverse effect when given a drug).

The most important of the genome projects, the complete sequence of the human genome, has recently been finished. This project revealed the complete sequence of the 3 billion bases and the relative positions of all estimated 30-40.000 genes in this genome. The genes are translated into a far larger number of proteins for example by differential splicing, and the proteins can in addition be post-translationally modified, for example by the formation of disulfide bridges between cysteins or phosphorylation of amino acid side chains. Additionally protein expression can be up- and down-regulated depending on the status of a cell.

Further variations of proteins can be expected based on DNA sequence variations from one individual to the next. One branch of genomics termed genotyping is focusing on the assessment of genomic sequence variation for the attribution of causative gene variants. Genomic sequence is static and thus does not allow the determination of the point of onset of a genetic disease without knowing the real correlations in a cell.

The analysis of levels of expression of RNA transcripts is more indicative. Up-and down regulation of mRNA matrices for protein synthesis is detected giving an indirect hint about the protein level in the cell. Although quantification can be done the real quantity of proteins remains uncertain and secondary modifications cannot be elucidated. Further, RNA is less stable than DNA and thus more difficult to handle and normalization of RNA levels does pose problems. Oligonucleotide arrays have reached great popularity for expression analysis (Duggan et al. Nat.Genet. 21 (Suppl.), 10-13 (1999)). The RNA pool of control cells is tagged with one fluorescent dye, while the RNA pool of cells deriving from cases is tagged with a different dye. Both pools are simultaneously hybridized to one array and by comparison of the emitted fluorescence of the two dyes quantification is achieved. A number of review articles dealing with RNA and array technologies in general were published in a supplement of Nature Genetics in January 1999.

Moving to next level, the study of all proteins of a cell termed proteomics has reached great popularity because it directly analyses the protein status and thus the active components of a cell. A proteome has been defined as the protein complement expressed by the genome of a cell or an organism. Although the real problems might be tackled by proteomics suitable methods that give a global high resolution overview are currently not available. Additionally to secondary modifications the most interesting processes are at low-level regulation of gene expression and are linked to changes from no copy per cell to very few copies. Both on the RNA and protein level these are currently hard if not impossible to detect.

Proteomics has mainly been advanced through the application of mass spectrometry (Karas and Hillenkamp, Anal. Chem. 60, 2299-2301 (1988); Fenn et al., Science 246, 64-71 (1989)). In proteomics matrix-assisted laser desorption/ionization mass spectrometry (MALDI) is used to analyze the product mixture of proteins digested by trypsin. The detected masses give a fingerprint that on comparison with a database allows the identification of the proteins.

When the fingerprint of the protein is not known, it remains possible to identify the protein by sequencing the different peptides, for example using Election Spray Ionization mass spectrometry (ESI), or through conventional methods. Digestion of the protein by various proteases followed by identification of the mass and sequence of the peptides allows the determination of the whole sequence of the protein. These methods are well known by the person skilled in the art (Jenkins and Pennington (Proteomics 1, 13-29 (2001), Siuzak (Proc Natl Acad Sci USA 1994 ; 91(24):11290-7)).

However, great interest lies in an extensive analysis of proteins contained in a sample, in particular a biological sample, for example a bodily fluid, or a sample harvested from a particular organ, especially a tumor. Several tens of thousands or even hundreds of thousands of proteins could be in such a fluid, or organ.

It is also interesting to perform comparative analysis between two or more samples, in order to determine the difference in the protein contents between said samples, especially when one sample is originating from normal state and another is originating from a pathological state. Comparison of the proteins within these two samples would give a protein fingerprint that is specific of the studied pathological state. This fingerprint could thus be used in a diagnosis process, and may allow early identification of the pathology before apparition of the clinical signs.

Comparative analysis may also be very interesting in order to determine the responsiveness of a target patient to a test or treatment. This would allow to better adapt the treatments to the patient, something extremely interesting in cancer cure. For example, an analysis is performed in order to identify the proteins that are qualitatively or quantitatively differentially expressed following treatment of a patient responsive to said test or treatment, using the method of the invention, as will be described below. A fingerprint of "responsiveness to treatment" is thus obtained. Then, an analysis of biological samples issued from said target patient before and after start of the test or treatment is then performed, and the match to the fingerprint allows to deduce the responsiveness of said target patient to said test or treatment by the presence of said proteins identified in the first step.

In practice of the state-of-the-art extracted proteins are separated on two-dimensional gels. The first separation dimension is achieved by isoelectric focussing with a pH gradient, the second by size (Klose Methods Mol. Biol. 112, 147-172 (1999)). The gel is then stained with Coomassie or silver. Usually the detection sensitivity allows the identification of a few thousand spots per gel. Spots are excised, digested with a protease (e.g. trypsin) and analysed by MALDI (Karas and Hillenkamp Anal. Chem. 60, 2299-2301 (1988)). In order to increase the efficiency of the tryptic digestion, processing of samples in very small volumes can be done (Eickhoff et al. WO 01/26797 A2). Further information about the peptides can be obtained by sequencing using the post source decay mode of a MALDI mass spectrometer, or ESI mass spectrometer.

Unfortunately the detection thresholds of the gel-staining methods do not allow the detection of a large part of the proteins present and low level proteins are masked by proteins of high abundance. Only several thousand proteins are usually identified on a gel, which probably represents only a few percent of the total proteins present. The majority of detected proteins are housekeeping genes with little diagnostic interest and impact. Additionally as the proteins are only separated by two properties (size and pI) the resolution of 2-D gels is not high enough. With 2-D gels quantification is practically impossible. Analysis by means of 2-D gels is feasible particularly for proteins of a size from 30kD-80kD. Another major drawback is that only soluble proteins can be separated with gels. Membrane proteins that are of greatest physiological interest are impossible to separate on gels. A comprehensive review is given by Jenkins and Pennington (Proteomics 1, 13-29 (2001)).

A complementary technique of 2-D gels termed SELDI (surface enhanced laser desorption/ionisation) was developed recently (US 5719060, US 6020208; EP0990256; EP0990257; EP0990258). The procedure is based on chromatographic procedures followed by mass spectrometric analysis (Siegel J. Mass Spectrom. 33, 264-273 (1998); Washburn Nat. Biotechnol. 242-247 (2001)). Thereby proteins of a mixture are bound to several unspecific features including hydrophobic surfaces, ionic surfaces etc. and subsequently washed using different conditions so that only a part of the proteins remains on a feature. By this procedure the complexity of a mixture is reduced as proteins are allocated to a certain position. For MALDI analysis matrix is applied onto the features and the proteins are analyzed in a time-of-flight mass spectrometer. This method is feasible for small proteins not exceeding a mass range of 30 kD. It is the belief of the authors that quantification is not feasible by SELDI as generally only small and broad signal peaks are obtained. Secondary modifications cannot be analyzed.

2-D gels or SELDI are complementary techniques concerning the size of the proteins with the advantage of SELDI being more powerful as more than two dimensions are used for separation of proteins (WO 98/59360; WO 00/66265; WO 00/67293). However both methods are not apt for quantification and as whole proteins are measured the resolution of signals is not high. An unambiguous identification of proteins is impossible. Furthermore the analysis of secondary modifications of all proteins on a sample is difficult if not impossible.

Several procedures for the quantification of proteins have been described (Zhou et al. Nat. Biotechnol. 19, 375-378 (2001); Oda et al. Nat. Biotechnol. 19, 379-382 (2001); WO 00/112208). However, these methods are not suitable for a survey of the complete protein load of a cell. They were rather developed to enrich certain proteins. Specific reagents containing a reactive group to tag defined chemical functions of an amino acid of peptides deriving from a tryptic digestion are used. The tags contain a linker and a binding group, generally consisting of biotin, that can be used for separation on streptavidin-coated magnetic beads. The linker is further used for introducing isotopic labels. Similar to RNA quantification, proteins captured of control cells are tagged with a molecule containing a linker with one kind of isotope, while the proteins of cells derived from cases are tagged with molecules containing another isotope. As two fluorescent emissions are used for quantification of RNA arrays, the signal intensities of corresponding peptides or proteins in the mass spectrum are compared for quantification, using additionally internal and external standards. In a variant of this approach, control and case cells are fed with different isotopes of nitrogen, so that the proteins of case and control cells are distinguishable by comparison of signal heights in mass spectra (Oda et al. Proc. Natl. Acad. Sci. USA, 96, 6591-6596 (1999)).

An alternative method for protein quantification could be the use of protein arrays and detection and quantification of binding using surface plasmon resonance (SPR) analysis. This is an optical detection system that was developed recently by Biacore (www.biacore.com). This method is also described in combination with arrays of chemical libraries (DE 19923820; DE 10008006; DE 19920156). This method has also been used in conjunction with subsequent mass spectrometric analysis of affinity bound samples (Nedelkov and Nelson, International Laboratory, 31 (6), September, 8-15 (2001).

A further method for quantification uses a luminescent or radioactive substance, an enzyme or a metal containing substance for quantification of antibodies or antigens (US 4020151).

Electrospray ionization mass spectrometry (ESI) is another method used to characterize proteins (Fenn et al. Science 246, 64-71 (1989)). In general recording a spectrum in ESI is slower than MALDI, yet gives higher resolution. Like MALDI, ESI can be used to generate sequence information of peptides. Peptides are sequenced by using the collision induced fragmentation of the peptides in the mass spectrometer.

Apart from these two ionization methods (MALDI and ESI) for volatilization of biomolecules, huge progress has been made in recent years in terms of separation of ions and analysis thereof in the mass spectrometer. The main developments were made in the use of alternative ion extraction procedures in MALDI, and the applications of quadrupols and ion traps to isolate specific ions in ESI, reflectrons to increase resolution and the usage of orthogonal set-ups to pulse ion packages into the mass spectrometer. State-of-the-art mass spectrometric analysis allows virtually any combination of MALDI and ESI with any separation and analysis method (reflectrons, time-of-flight analysis,...).

By fabricating microarrays of small molecules (prepared by split-and-pool synthesis), large libraries of compounds can be screened very efficiently to identify new ligands for virtually any protein of interest (Schreiber Science 17, 1964-1969, (2000)). Such ligands can then be used to study the biological role of its protein target by perturbing its function in vivo.

Protein arrays are becoming a reality (WO 00/54046). Recent advances in protein array technology are described in the several publications (Kodadek, Chem. Biol. 8, 105-115 (2001); Haab et al., Genome Biology 2, 0004.1-0004.13 (2001); Zhu and Snyder, Current Opinion in Chemical Biology, 5, 40-45 (2001); Fields, Science 291, 1221-1224 (2001); MacBeath and Schneider, Science 289, 1760-1763 (2000); Emili and Cagney, Nature Biotechnology 18, 393-397 (2000); Walter et al., Current Opinion in Microbiology 3, 298-302 (2000); Arenkov et al., Analytical Biochemistry 278, 123-131 (2000); Holt et al., Current Opinion in Biotechnology 11, 445-449 (2000); Roda et al., Biotechniques 28, 492-496 (2000); Lueking et al., Analytical Biochemistry 270, 103-111 (1999); WO 00/29444; WO 00/04382).

For protein arrays, proteins are expressed and attached to a surface of a glass slide or other support in an arrayed pattern. In conjunction with high throughput expression and purification of recombinant proteins, microarrays of functionally active proteins were prepared on glass slides. These arrays are then used to identify protein-protein interactions, to identify, for example, the substrates of protein kinases, or to identify the targets of biologically active small molecules.

While transcriptional profiling provides invaluable insight into biological function on a genome-wide scale, it does not offer information on regulation that occurs at the protein level (e.g., degradation, phosphorylation/dephosphorylation, sub-cellular localisation, etc.). The possibility of using microarrays of antibodies to study regulation at the protein level is under investigation (de Wildt et al. Nat. Biotechnol. 18, 989-994 (2000)). Polyclonal antibodies can be produced by initiating an immunological reaction of an animal caused by high abundance of the protein of interest.

In principle, libraries of proteins or peptides deriving from phage display, ribosome display or any other method to create libraries of proteins and peptides can be spotted onto a surface for subsequent binding of proteins (Li et al. Nat. Biotechnol. 18, 1251-1256 (2000); Kay et al. Methods 24, 240-246 (2001); Holt et al. Curr. Opin. Biotechnol., 11, 445-449 (2000)).

The construction of arrays by molecule libraries is not restricted to poly amino acids or organic molecules but can also be done by nucleic acids such as RNAs analogously to RNA arrays. However, the generation of specific addresses for protein binding is not that easy. Nucleic acids on RNA expression arrays bind, obeying the rules of Watson-Crick base-pairing. For specific protein binding suitable RNAs have to be found out in a selection process termed SELEX (Sun Curr. Opin. Mol. Ther., 100-105 (2000); Jayasena Clin. Chem. 1628-1650 (1999); Doi and Yanagawa Comb. Chem. High Throughput Screen 4,497-509 (2001), WO 99/27133).

Gel-pad based microarrays are described in several publications (US 5981734; US 6143499; US 5770721; US 5756050)

Nanoelectrode arrays are also a possible solution for separation of protein mixtures on a chip. Three-dimensional electrochemical binding profiles, which mimic traditional chemical binding sites, are applied (US 6123819) to capture specifically a protein.

Other relevant publications to the state-of-the-art are WO 00/61806; WO 00/54046; US 4020151).

The state-of-the-art of protein chemistry and methods for protein analysis is described in "Proteome and protein analysis", ed. Kamp, Springer Verlag, ISBN 3-540-65891-2 (2000) and "Proteins Labfax", ed. Price, BIOS Scientific Publishers, ISBN 0-12-564710-7 (1996).

Proteomics (systematic analysis of proteins) suffers from the severe limitation that with 2-D gel analysis and subsequent mass spectrometric analysis of tryptic digest products only very abundant proteins, that are of limited interest, can be analysed. Protein arrays on the other hand, at the current state-of-the-art, are difficult to produce with high variability and resolution. Currently no method exists to generate a protein array with high variant coverage, a possibility of normalizing it, a method to analyze it with high resolution, thus providing a method for high resolution, selective protein analysis which can also be applied to the analysis of low abundance proteins. Another major problem of protein analysis is, that no possibility exists to analyze two or more complete protein extracts simultaneously on one analysis device, thus eliminating the variability between two analysis devices.

In contrast to expression profiling the reaction sequence of a state-of-the-art protein analysis experiment lays itself significantly more open to experimental variation and two situations are never directly comparable. In protein analysis, two protein extracts are not analyzed in the same experiment at the same time. Different samples are dealt with sequentially.

The invention provides a method of analysis proteins that may be used to allow simultaneous analysis of two or more complete protein extracts on the same analysis platform, with complete resolution of relative protein identities, as well as analysis of post-translational modifications and quantities.

This invention relates to a method for protein analysis. The operating medium of the method is a capture array. This capture assay provides a means for stratification of the protein extract (separation of the proteins according to some of their structural features) and later a support for the subsequent treatment.

Thus, the invention relates to a method for identifying and/or quantifying and/or characterizing multiple proteins in a sample containing proteins, said method comprising:
a) optionally labeling the proteins in the sample with a marker,
b) bringing the proteins into contact with an array comprising one or more feature(s), leading to specific capture of different proteins on different feature(s) on the array,
c) applying, to the protein(s) captured on the feature(s) of the array, a procedure giving a fingerprint specific of the protein(s) on the feature(s), the comparison of the data obtained for the protein(s) on the feature(s) with a fingerprint library (database) allowing identification and/or quantification and/or characterization of the proteins present in the sample, including the post-translational modifications.

In a specific embodiment of the invention, the composition or sequence of the proteins in said biological sample may be at least partially unknown, and the identification and the characterization of the unknown protein that can not be performed by comparison with databases is performed by further sequencing of the proteins or peptides, in particular by mass spectrometry.

Preferably, the capture of the proteins by the feature(s) on the array depends on the structure of the proteins, in particular the primary structure of the protein (sequence of the protein), but preferably on the 3-dimentional structure of the protein.

In a specific embodiment, the quantification of the proteins is performed by adding, to the sample, a specific and quantified protein as a standard, the quantification of the proteins in the sample being calculated by comparison with the standard. The absolute quantification of the proteins is obtained from their relative weight with regard to the quantity of the standard protein. It is calculated from the intensity of the signals obtained, constitutive of the fingerprint.

In the most preferred embodiment, the fingerprint is peptide-based.

Preferably, in this embodiment, the proteins are captured on the array, which allows stratifying the proteins from the starting protein mixture. The procedure that is subsequently applied, in order to obtain the fingerprint useful for the subsequent simultaneous identification and quantification of the proteins comprises of breaking down the captured proteins into specific peptide fragments on the feature preferably followed by identification of the proteins by their peptide fingerprints by mass spectrometry. The breaking down of the proteins into specific peptides is preferably performed by digestion with a specific enzyme such as trypsin, that cuts the proteins at specific and well known amino acids. Starting from the protein databases such as the one on the NCBI web site (http://www.ncbi.nlm.nih.gov) or such as SwissProt, or the EMBL database, it is easy to simulate digests of proteins with trypsin, and build a database linking trypsin-digest peptides and proteins. Software exists that simulate digests of proteins by various proteolytic enzymes or reagent cleavage (http://bioweb.pasteur.fr/seqanal/interfaces/digest.html). Databases exist that integrate whole sequence DNA translated into theoretical protein peptides fingerprints, such as Mowse or Mascot, distributed by Matrix Science (London, UK, www.matrixscience.com)

Starting the expected fingerprint obtained from the analysis of the databases, it is possible to determine post-translational modifications, if some are present. Indeed, the theoretical mass of some peptides may be calculated if no post-translational modifications are present., and any differences between the theoretical mass and the observed mass implies the presence of a moiety on the peptide. The mass of said moiety is easily calculated and the use of databases recording the characteristics of the most common post-translational moieties (for example glycosylation, or phosphorylation) may allow to determine its nature.

The quantity of the proteins on the feature on the array are determined either on an relative scale (one compared to the other), or may be absolute, with the help of a standard peptide. Mass spectrometry indeed allows to correlate the quantity of a peptide and the intensity of the peak corresponding to said peptide.

Thus, it is possible to compare different peptide fingerprints from different features of the array and/or to compare peptides on one feature, by comparing their peak intensity.

Both measurements (qualitative and quantitative) are preferably achieved by mass spectrometric analysis.

The invention also allows the analysis and comparison of two or more protein samples in a single procedure. The invention relates to a method for identifying proteins that are qualitatively or quantitatively differentially expressed between at least two biological samples containing proteins, said method comprising:
a) labeling the proteins in each sample with a different marker, with the optional possibility of not labeling the proteins in one sample,
b) mixing together the proteins of all different samples and bringing the mixture into contact with an array comprising one or more feature(s), leading to specific capture of different proteins on different feature(s) on the array,
c) applying, to the proteins captured on the feature(s) of the array, a procedure allowing identification/response of the markers, the differences in the data obtained for each marker allowing the identification of the proteins that are qualitatively and/or quantitatively differentially expressed between the different samples.

In a specific embodiment, the composition or sequence of some of the proteins in said biological samples may be at least partially unknown. The method of the invention allows nevertheless to determine that these unknown proteins are differentially expressed (quantitatively or qualitatively), and the final identification of the unknown proteins may be performed by sequencing of the proteins, in particular by mass spectrometry.

The method can be used to assay exactly two different samples, or more than two different samples.

In the preferred embodiment, the procedure allowing identification/response of the different markers used for the different samples comprises the digestion of the proteins on the feature(s), in particular by adding a protease or a cleavage reagent to the feature(s) of the array, giving a digest mixture of the protein(s) that are localized on said feature.

In the preferred embodiment, said procedure comprises analysis of said digest mixture by mass spectrometry, and in particular, matrix-assisted laser desorption/ionization is used to transfer the peptides into the mass spectrometer.

The figures in the application explain the principle of the invention. When different tags are used on the different samples (and it is possible not to use a tag on one of the samples, when mass spectrometry is performed), the relative abundance of the tags is used for analysis of the relative abundance of the proteins.

When mass spectrometry is used, different mass tags (described below) are used in the different samples. Upon digest of the proteins by proteases or cleavage reagents, some peptides will be labeled by the mass tag, while others will not (for example, if a mass tag specific of the N-terminus of the protein is used, only the N-terminal peptide will be labeled). Analysis by mass spectrometry will then lead to a spectrum such that the unmarked peptides originating from the proteins of all samples will lead to a single peak, while a discrimination will be observed for the labeled peptides, the increment between the peaks being equal to the difference in the mass tags.

Analysis of the difference in the intensity of the peaks gives an immediate knowledge of the relative abundance of the target protein in each sample. By an abuse of language, the "intensity of the peaks specific of each mass tag marker" may be called "the intensity of the marker".

In a preferred embodiment the proteins of a protein mixture or extract are stratified on a capture array by binding to structural elements (features). These features are preferably attached to the surface of a carrier that can be brought into contact with the full protein extract. Preferably the features are made up of molecules or combinations of the molecules of the following list: nucleic acids, oligonucleotides, oligopeptides, polypeptides, antibodies, oligosaccharides, polysaccharides, organic molecules, polymers and inorganic molecules. In order to generate a diverse library of features different approaches may be applied. Features are applied to significantly reduce the complexity of a protein mixture by binding only one or a few proteins per feature. In contrast to the major part of array technologies no selective binding (one feature one protein) is required.

In the case of nucleic acid arrays the following strategy for the generation of diversity is preferably adopted. A DNA sequence, for example, that is known to form a stable 3-dimensional structure is used as a starting point. This DNA sequence is amplified by PCR in a manner that results in the original sequence being mutated and therefore new 3-dimensional structures. These procedures are called error-prone PCR (Wang et al. J. Comput. Biol. 7, 143-158, 2000; Cherry et al. Nat. Biotechnol. 17, 333-334, 1999). Another strategy makes use of imitating natural recombination and DNA (exon) shuffling (Volkov and Arnold Methods Enzymol. 328, 447-456, 2000; Petrounia and Arnold Curr. Opin. Biotechnol. 11, 325-330, 2000; Kolkman and Stemmer Nat. Biotechnol. 19, 423-428, 2001; Minshull and Stemmer Curr. Opin. Chem. Biol. 3, 284-290, 1999; Crameri et al. Nat. Med. 2, 100-102, 1996). To introduce mutations the PCR can be starved of a required building block (one of the four dNTPs), which results in errors of the DNA polymerase, or by introducing a variant of a DNA building block that can act as a substitute for several of the bases. This way random sequences starting from defined sequence are generated. Finally, the mutated PCR product is cloned into a vector, used for transfection and grown on a culture. The different colonies are picked, DNA harvested and the inserts amplified by PCR. Each colony gives rise to a feature on the array. The PCR products are spotted onto a carrier in an arrayed structure.

RNA molecules can also be made by RT-PCR to the same end, for example, by a procedure called SELEX (Systematic Evolution of Ligands by EXponential enrichment, Tuerk and Gold, Science 249, 505-510, (1990)). This procedure involves cycles of affinity selection by a target molecule from a heterogeneous population of nucleic acids, replication of the bound species (the ligands), and in vitro transcription to generate an enriched pool of RNA. Binding RNAs are also termed aptamers, which have a defined 3-dimensional structure. In parting from the strategy of generation of aptamers we accept the unselected library to generate the diverse structures of our array. Similarly the molecules take unique and defined structures. As said aptamers are selected to accommodate a certain structure and thus be specific for a particular interaction partner. Here it is sought that the structures of the features are generated to contain a degree of randomness.

In the case of the array of features being made up of proteins an approach similar to Affibody (www.affibody.com) is applied, except that, in this case, it is preferred if the structures are not selected in a certain direction, but randomly mutated away from a defined structure (Hansson et al,, Immunotechnology, 4, 237-252 (1999); Gunneriusson et al., Protein Engineering, 12, 872-878 (1999)). By injection of a mouse with a protein a large amount of polyclonal antibodies is generated in the animal. These antibodies can be isolated for use as a binding component of the respective protein. No monoclonal antibodies are needed, as only semi-specific binding is desired.

In the case of the later described sequence specific protease digestion of the captured protein, specific digestion of capture features will also occur. The peaks resulting from the features are subtracted from the mass spectra for the characterization of the captured proteins. These peaks can also serve to assess the quality of the individual feature of the array or the quality of the reactions carried out on the feature. In the case of protein arrays the randomized sequences can also be cloned into an expression vector and allowed to express protein. The individual clones are spotted onto the carrier in an arrayed format.

In a preferred embodiment, said features are immobilized on a surface made of materials such as glass, silicate, metal, metal-coated glass, glass-coated metal or plastic. The methods that lend themselves for immobilization are of the following: binding via NH₂, I, SH, N-hydroxy-succinimide, biotin, His6 or other. The coating of the carrier material can be NH₂, I, SH, N-hydroxy-succinimide, streptavidin, Ni or any other specifically interacting chemical group with the functionality of the feature. Immobilization can rely on covalent binding of the substrate on the surface or other strong interaction that can withstand subsequent reactions on the array. In another preferred embodiment the features are captured inside pores of a substrate. These substrates could be a membrane (Nylon, PVDF) or a gel pad (agarose, polyacrylamide). The features can be covalently bound to the porous material. This can be achieved by photochemical or chemical cross-linking. Alternatively, hydrophobic interaction can serve to immobilize the features. This support of the features has the advantage that, in contrast to the 2-dimensional surface of for example a glass slide, it is more amenable to maintaining the 3-dimensional structure of the feature. Not bound features are removed by washing.

An important and preferred characteristic of each of the arrayed features is that a specific protein or a specific group of proteins has affinity, preferably high affinity for it. Thus the entire protein extract is split into fractions (stratification) on the different features of the array.

The present invention relates to the identification and quantification of the captured proteins on each feature and the use of proteins captured on several features to assess quantities and post-translational modifications. The final objective is to create an array capable of capturing all proteins of the proteome of a cell on defined positions for comparative analysis of proteins, such as identities, post-translational modifications and quantities.

Thus, the array that is being used in the invention preferably comprises a number of diverse features high enough to bind the proteins in the extract. Different arrays may be used if the aim is to study specific proteins or specific types of proteins (nucleic acid-binding proteins, membrane proteins, antigen-binding proteins...).

The identification (qualitative characterization) of the captured proteins is preferably achieved by a method, comprising the generation of a peptide fingerprint of the proteins captured immediately on the feature by mass spectrometry.

This is achieved by digesting captured proteins on the feature with a sequence specific protease, or a cleavage reagent and mass analyzing the peptide fragments. By comparison with databases the peptide mass fingerprint suffices to identify the proteins the peptide fragments originate from (trypsin is the protease most frequently for this purpose). Alternatively, CNBr cleavage, which cuts at methionine can be applied. Other reagents include Lys-C, Arg-C, Asp-N, V8-bicarb, V8-phosp, chymotrypsin.

As explained above, post-translational modifications of the proteins can be identified by mass shift of expected peptide mass in the peptide mass fingerprints.

In the case of a mixture of several proteins the compounded peptide mass fingerprint can be deconvoluted to identify the proteins contained in the mixture. The current state-of-the-art allows deconvoluting mixtures of up to 20 proteins from peptide mass fingerprints. With the improvement of mass spectrometric methods this number is likely to increase.

In the case of an array of features that are of protein nature, the feature will contribute peptides to the mass spectrometric analysis. They are subtracted from the mass list for database comparison, thus only leaving the remaining peaks as being attributed to captured proteins.

In a preferred embodiment of the invention matrix-assisted laser desorption/ionization is used to transfer the peptides of a feature into the mass spectrometer. This can be done by directly introducing the array, thus not transferring the samples prepared on the array. An advantage of this is that there is no loss of material for the analysis and more importantly no selective loss. Typically, a matrix has to be added prior to the introduction of the samples into the mass spectrometer. A preferred matrix for this is α-cyano-4-hydroxy-cinnamic acid, but also other matrices could be used. State-of-the-art MALDI mass spectrometers allow sizing of desorption products as well as the analysis of post-source-decay (the spontaneous fragmentation of the peptide bonds after the desorption process, which allows the determination of the amino acid sequence of the peptide). Analysis of post-source decay may be needed when a peptide fingerprint can not be assigned to a protein present in a database (for example if the protein is not known). Obtaining the sequence of the different peptides will allow to reconstitute the whole sequence of the protein. This may require performing two analysis, using two different proteases, in order to speed up the process of reconstituting the whole protein sequence. The person skilled in the art is aware of the techniques to use for applying mass spectrometry to obtain the sequence of an unknown protein.

Alternatively, other mass spectrometers, like electrospray instruments can also be applied. For this the individual sample may have to be eluted from the feature and transferred into the mass spectrometer. An advantage of using this sort of mass spectrometer (especially ESI) is that they provide significantly higher resolution and that they are routinely coupled with sector analysis. Therefore, breaks can be actively introduced by bleeding a fragmentation gas into the mass spectrometer. This provides an active rather than a passive means for peptide sequencing.

The method of the invention is particularly performed with identification of the masses of the peptides by time-of-flight or magnetic field deviation analysis in an ion trap or quadrupol.

As previously said, it is advantageously completed by further characterization of the peptides in said digest mixture for sequence elements by analysis of post source decay products, if needed, in particular if the step of comparison of peptide masses to a protein database to identify the proteins that were bound to the feature and to identify post-translational modifications of the proteins does not prove successful (for example Mowse and Mascot, *op.cit.*).

In a particularly preferred embodiment of this invention two or more protein extracts are compared to each other. In order to distinguish the two or more protein extractions at least one of the protein extracts is subjected to a modification chemistry that results in defined modifications of chemical functions of the proteins.

A preferred modification is such as not to alter the 3-dimensional structure of the protein. A way to achieve this may be by attaching a chemical group by trinitrobenzene sulfonic acid, ethylthiofluoro acetate, succinic anhydride, phenylisothiocyanate, Dansyl chloride, acetic anhydride, polyethylene glycol, or similar reagents to the (deprotected) N-terminal group of the protein. As the N-termini of proteins are frequently blocked, it can be necessary to cleave off these blocking groups prior to N-terminal modification. Methods for this are described by Kamp and Hirano (Chapter 22, Proteome and Protein Analysis, ed. Kamp, Springer Verlag, 2000).

Different protein extracts would, for example, be tagged with different mass-shifting molecules. Thereafter comparing correlatable N-terminal peptides, stemming from different protein extracts, the abundance of a particular protein in the different protein extracts is measured.

For calibration of the entire array, the effects on proteins captured by different features are correlated with each other by a global analysis. This way one can determine which are the proteins which have changed, increased or decreased in different protein extractions.

It is also possible to modify several of the peptides of a protein, for example by NH₂-specific protein modification with trinitrobenzene sulfonic acid, ethylthiofluoro acetate, succinic anhydride, phenylisothiocyanate, Dansyl chloride, acetic anhydride, attachment of polyethylene glycol or similar reagents. Also SH-specific protein modifications with β-mercatoethanol, dithiothreitol, iodoacetic acid, iodoacetamide or similar reagents is possible. For chemically modifying carboxyl groups of proteins full or limited amidation by 1-ethyl-3-[3-(dimethylamino)propyl]carboiimide hydrochloride (EDC) and an amine like glycine methyl ester, glycinamide, methylamine, ethanolamine or similar can be used. Tyrosines can be modified by nitration with tetranitromethane (also oxidation of thiols) and tryptophan can be modified by specific oxidation for example by N-bromosuccinimide or limited oxidation with ozone. Again different protein extracts are modified with similar compounds (same chemical functionalities, thus the chemical yield of reactions has less of an influence) but with different masses. Modifying compounds can be different by having additional methyl groups. Modifying chemical groups can be isotopically pure, which results in less broad peaks in the peptide mass fingerprints. For tagging different extracts similar chemical compounds with different isotope composition are employed. By these modifications several of the peptides of each can be drawn into the quantification procedure.

Protein mixes can either be stratified together or they can be applied to the array one by one. In this strategy the modification of each of the protein extraction can be carried out after the capture. This way the effect of the modification on the 3-dimensional structure can be reduced.

The method of the invention is preferably applied with an array that comprises multiple features allowing binding of the whole proteome of a target organism or a target cell, allowing subsequent identification and/or quantification and/or characterization of proteins comprised in said proteome.

For obtaining the quantification, a known protein (standard) may quantified by known methods, and the quantity of another target protein in the proteome is obtained by the relative intensity of the mass peaks of said target protein and said standard protein.

By analyzing the quantitative and qualitative differences in protein expression between at least two samples, the method and the array of the invention may be used for various applications, amongst which:
- A method for identifying compounds that interact with a selected target protein, comprising the steps of:
   a) applying said protein on an array comprising different features, said features comprising different compounds,
   b) selecting the compounds that interact with the selected target protein, by identifying the features to which is bound said protein.

   This method may be used in particular if the process of attaching the proteins on the array does not modify the 3-dimentional structure of the proteins. Rather than testing a multiplicity of compounds on a specific target, this method allows to test a multiplicity of compounds on a multiplicity of potential targets.
- A method for identifying proteins that are qualitatively or quantitatively differentially expressed between two different distinguishable phenotypes, (as defined above) comprising performing the method of the invention, on a first sample that is representative of a first phenotype and a second sample that is representative of a second distinguishable phenotype. In a specific embodiment, the first sample is harvested from a patient suffering of a disease, and the second sample is obtained from a patient who does not suffer from said disease. In a preferred embodiment, said two distinguishable phenotypes are tumor bearing patient and healthy patient.
- A method for identifying proteins that are qualitatively or quantitatively differentially expressed following treatment of a biological sample with a test compound, comprising performing the method of the invention on a first sample that is representative of said biological sample before treatment with said compound, and a second sample that is representative of said biological sample during or after treatment with said compound.
- A method of determining or assessing the therapeutic potential of a test compound with respect to a biological sample, comprising:
   a) performing the method as described above to said biological sample with a compound known to have therapeutic potential, in order to identify the proteins that are qualitatively or quantitatively differentially expressed following treatment of a biological sample with a compound with therapeutic potential,
   b) applying said test compound to said biological sample,
   c) deducing the therapeutic potential of said test compound by the expression profile of the proteins in the sample and/or presence of said proteins identified in step a).
- A method of determining or assessing the responsiveness of a target patient to a test or treatment, comprising:
   a) performing the method described above to a biological sample issued from a reference patient that is responsive to said test or treatment, in order to identify the proteins that are qualitatively or quantitatively differentially expressed in response to said test or treatment,
   b) performing the method of the invention, allowing comparison between two samples, to biological samples issued from said target patient before and after start of the test or treatment, deducing the responsiveness of said target patient to said test or treatment by the presence of said proteins identified in step a).

The invention described here will replace recent tools for proteomics for efficient study of protein expression. This can be done for example in microorganisms with the ultimate goal to engineer improved production strains for fine chemicals. Another application will be the comparative study of the proteome of cells with a normal phenotype and cells with an abnormal phenotype such as a disease. The gained information can be used to create targets for medication. The principle of the procedure would be similar for each kind of application.

It is the aim to establish protein arrays covering the whole proteome of a cell or a microorganism.

Therefore in a first step features are identified by one of the methods described above and positioned on a defined position on an array. For example, a large amount of features created by evolutional methods is displayed on an array. Binding of proteins on different features is analyzed by mass spectrometry. Features binding a certain number of proteins, which can be distinguished by the peptide patterns after trypsin digestion, are selected for the final analytic array. When the proteins binding to some features are not know, a further analysis (sequencing) is perform to determine their nature.

These chosen features address known proteins or proteins newly identified as is done with RNA arrays in order to reduce significantly the complexity of a protein mixture. It is optimal if approximately 10 different proteins bind per feature so that approximately 600 features are estimated to be sufficient to study the proteome of *E. coli*.

By the introduction of different mass tags, proteins originating from different samples can be distinguished in relation with their provenience and the relative quantity of said proteins can be analyzed by quantification of the height of mass spectrometric signals.

Additionally post-translational modifications like phosphorylation of a protein, the major tool of a cell for regulation, can be distinguished.

### Description of the figures

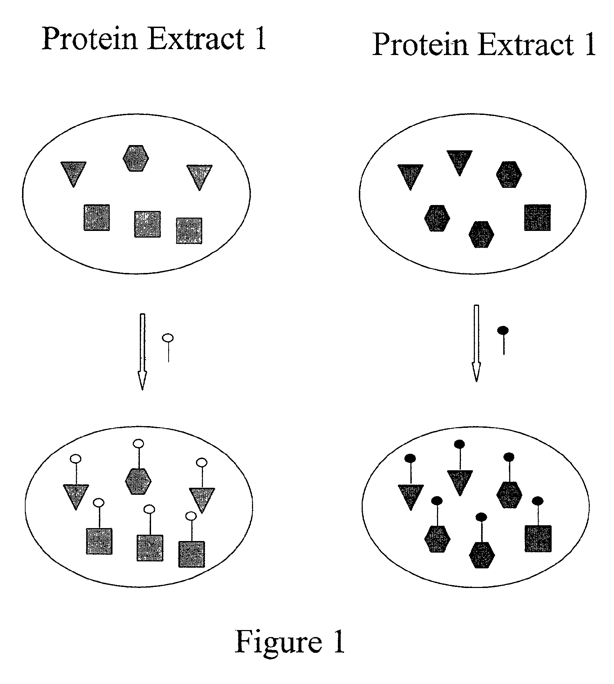

Figure 1 represents the labeling of two different protein extracts with a different mass tag.

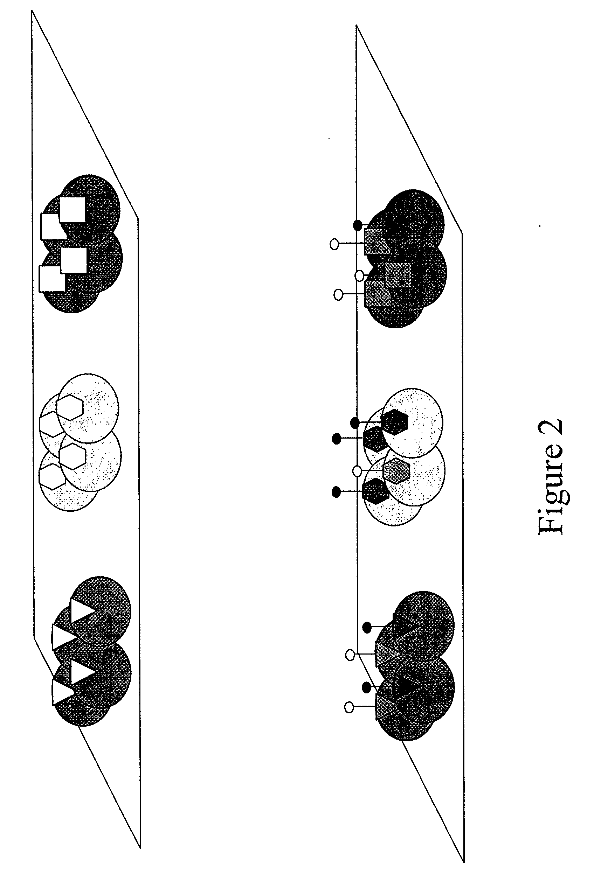

Figure 2 represents the stratification (discriminative binding) of the proteins of features on an array, that depends of the nature of the proteins.

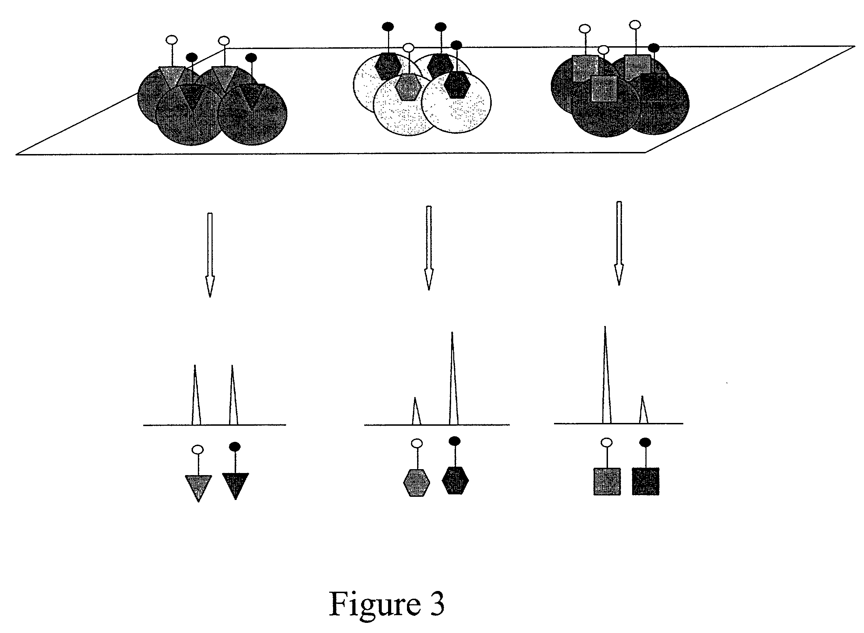

In Figure 3, mass analysis of the proteins on each feature is performed and the relative quantity of the proteins between the two samples is assessed by comparing the peak intensities.

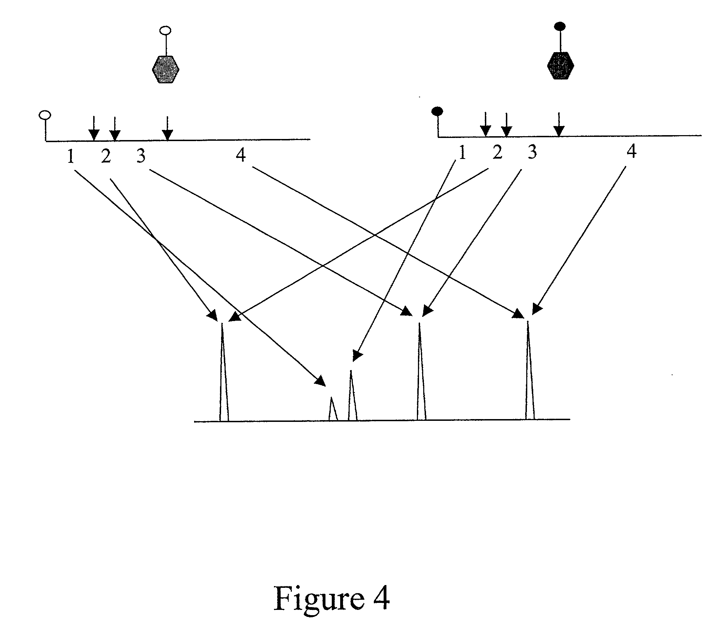

In Figure 4, mass spectrometry is performed after digest on the feature of the proteins. Only one peptide issued from the digest is labeled with a mass tag. The peptides that are not marked give the same peak for the proteins issued from the two samples, while a shift is observed for the labeled peptide, depending on the mass tag. Analysis of the intensity peak allows relative quantification of the proteins between the two samples.

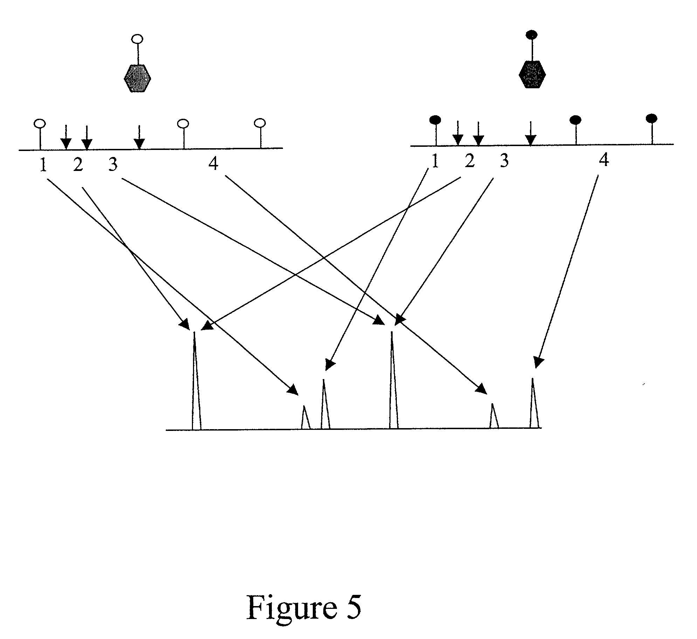

In Figure 5, the mass tags have been chosen such as being introduced in multiple sites in the proteins. On the four peptides obtained upon digest, two do not bear a mass tag, and they can not be separated by mass spectrometry, one does bear one mass tag, and separation occurs, and the last one bears two mass tags, and the discriminative gap is bigger than for the one that only bears one mass tag. Again, analysis of the intensity peak gives information about the relative quantities of the proteins between the two samples.

## Claims

1. A method for identifying and/or quantifying and/or characterizing multiple proteins in a sample containing proteins, said method comprising:
a) optionally labeling the proteins in the sample with a marker,
b) bringing the proteins into contact with an array comprising one or more feature(s), leading to specific capture of different proteins on different feature(s) on the array,
c) applying, to the protein(s) captured on the feature(s) of the array, a procedure giving a fingerprint of the protein(s) on the feature(s), the comparison of the data obtained for the protein(s) on the feature(s) with a fingerprint library allowing identification and/or quantification and/or characterization of the proteins present in the sample, including the post-translational modifications.

2. A method for identifying proteins that are qualitatively or quantitatively differentially expressed between at least two biological samples containing proteins, said method comprising:
a) labeling the proteins in each sample with a different marker, with the possibility of not labeling the proteins in one sample,
b) mixing together the proteins of all different samples and bringing the mixture into contact with an array comprising one or more feature(s), leading to specific capture of different proteins on different feature(s) on the array,
c) applying, to the proteins captured on the feature(s) of the array, a procedure allowing identification/response of the markers, the differences in the data obtained for each marker allowing the identification of the proteins that are qualitatively and/or quantitatively differentially expressed between the different samples.

3. The method of claim 1 or 2, wherein said features are chosen in the group consisting of nucleic acids, oligonucleotides, oligopeptides, polypeptides, antibodies, oligosaccharides, polysaccharides, organic molecules, polymers, inorganic molecules, and combination thereof.

4. The method of any of claims 1 to 3, wherein said features are immobilized on a surface.

5. The method of any of claims 1 to 4, wherein said array comprises a multiplicity of different features.

6. The method of any of claims 1 to 5, wherein said features are localized on said array such as to allow the identification of the address of each feature.

7. The method of any of claim 1 to 6, wherein each feature specifically interacts with one protein present in the protein mixture.

8. The method of any of claims 1 to 6, wherein at least one feature specifically interacts with more than one of the proteins present in the protein mixture, wherein said feature does not interact with all the proteins present in the protein mixture.

9. The method of any of claims 1 to 8, wherein said labeling of the proteins consists in a chemical modification of the proteins.

10. The method of claim 9, wherein said chemical modification does not alter the 3-dimensional structure of the protein.

11. The method of any of claims 9 to 10, wherein said chemical modification consists in attaching a chemical group chosen in the group consisting of trinitrobenzene sulfonic acid, ethylthiofluoro acetate, succinic anhydride, phenylisothiocyanate, Dansyl chloride, acetic anhydride, polyethylene glycol, and similar reagents to the (deprotected) N-terminal group of the protein.

12. The method of any of claims 9 to 10, wherein said chemical modification consists in inducing SH-specific protein modifications with an agent chosen in the group consisting of β-mercatoethanol, dithiothreitol, iodoacetic acid, iodoacetamide, and the like.

13. The method of any of claims 9 to 10, wherein said chemical modification consists in modifying carboxyl groups of proteins by full or limited amidation unsing an agent chosen in the group consisting of 1-ethyl-3-[3-(dimethylamino)propyl]carboiimide hydrochloride (EDC), an amine like glycine methyl ester, glycinamide, methylamine, ethanolamine and the like.

14. The method of any of claims 9 to 10, wherein said chemical modification consists in a modification of tyrosines by nitration with tetranitromethane (also oxidation of thiols), or of tryptophans by specific oxidation, for example by N-bromosuccinimide, or by limited oxidation with ozone.

15. The method of any of claims 1 to 14, wherein the procedure allowing identification/response of the marker(s) comprises adding a protease to the feature(s) of the array, giving a digest mixture of the protein(s) that are localized on said feature.

16. The method of claim 15, wherein said procedure comprises analysis of said digest mixture by mass spectrometry.

17. The method of claim 16, wherein matrix-assisted laser desorption/ionization or electrospray/ionization is used to transfer the peptides into the mass spectrometer.

18. The method of claim 17, wherein a matrix or a solvent is added to each feature for matrix-assisted laser desorption/ionization or electrospray/ionization.

19. The method of claim 16, wherein the peptides are transferred to an analysis array.

20. The method of any of claims 16 to 19, wherein the masses of the peptides are identified by time-of-flight or magnetic field deviation analysis in an ion trap or quadrupol.

21. The method of any of claims 16 to 20, further comprising characterization of the peptides in said digest mixture for sequence elements by analysis of post source decay products.

22. The method of any of claims 16 to 21, wherein peptide masses are compared to a protein database to identify the proteins that were bound to the feature and to identify post-translational modifications of the proteins.

23. The method of claim 2, wherein the intensity of each marker is used for protein quantification between the two protein samples.

24. The method of claim 2, wherein exactly two different samples are assayed.

25. The method of claim 2, wherein more than two different samples are assayed.

26. The method of claim 1, wherein said array comprises multiple features on allowing binding of the whole proteome of a target organism or a target cell, allowing subsequent identification and/or quantification and/or characterization of proteins comprised in said proteome.

27. A method for identifying compounds that interact with a selected target protein, comprising the steps of:
a) applying said protein to an array comprising different features, said features comprising different compounds,
b) selecting the compounds that interact with the selected target protein, by identifying the features to which is bound said protein.

28. A method for identifying proteins that are qualitatively or quantitatively differentially expressed between two different distinguishable phenotypes, comprising the step of performing the method of claim 2, on a first sample that is representative of a first phenotype and a second sample that is representative of a second distinguishable phenotype.

29. A method for identifying proteins that are qualitatively or quantitatively differentially expressed following treatment of a biological sample with a test compound, comprising the step of performing the method of claim 2 on a first sample that is representative of said biological sample before treatment with said compound, and a second sample that is representative of said biological sample during or after treatment with said compound.

30. A method of determining or assessing the therapeutic potential of a test compound with respect to a biological sample, comprising the steps of:
a) performing the method of claim 29 to said biological sample with a compound known to have therapeutic potential, in order to identify the proteins that are qualitatively or quantitatively differentially expressed following treatment of a biological sample with a compound with therapeutic potential,
b) applying said test compound to said biological sample,
c) deducing the therapeutic potential of said test compound by the presence of said proteins identified in step a).

31. A method of determining or assessing the responsiveness of a patient to a test or treatment, comprising the steps of:
a) performing the method of claim 29 to a biological sample issued from a patient that is responsive to said test or treatment, in order to identify the proteins that are qualitatively or quantitatively differentially expressed in response to said test or treatment,
b) performing the method of claim 2 to biological samples issued from said patient before and after start of the test or treatment, deducing the responsiveness of said patient to said test or treatment by the presence of said proteins identified in step a).
